(19) Europäisches Patentamt
European Patent Office
Office européen des brevets

(11) Veröffentlichungsnummer: **0 387 499 B1**

# (12) EUROPÄISCHE PATENTSCHRIFT

(45) Veröffentlichungstag der Patentschrift: **22.12.93**

(51) Int. Cl.[5]: **C07C 251/40**, C07C 251/52, A01N 37/50

(21) Anmeldenummer: **90101173.4**

(22) Anmeldetag: **20.01.90**

## (54) Substituierte Oximether sowie deren Verwendung als Schädlingsbekämpfungsmittel.

(30) Priorität: **16.02.89 DE 3904693**

(43) Veröffentlichungstag der Anmeldung:
**19.09.90 Patentblatt 90/38**

(45) Bekanntmachung des Hinweises auf die Patenterteilung:
**22.12.93 Patentblatt 93/51**

(84) Benannte Vertragsstaaten:
**BE CH DE DK FR GB IT LI NL**

(56) Entgegenhaltungen:
**EP-A- 0 088 326**
**EP-A- 0 178 826**
**DE-A- 2 428 070**
**GB-A- 2 029 223**

(73) Patentinhaber: **BAYER AG**

**D-51368 Leverkusen(DE)**

(72) Erfinder: **Gayer, Herbert, Dr.**
**Alfred-Delp-Strasse 4**
**D-4019 Monheim-Baumberg(DE)**
Erfinder: **Krämer, Wolfgang, Dr.**
**Rosenkranz 25**
**D-5093 Burscheid 2(DE)**
Erfinder: **Dutzmann, Stefan, Dr.**
**Leinenweberweg 33**
**D-4000 Düsseldorf 13(DE)**

**EP 0 387 499 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue subtituierte Oximether, mehrere Verfahren zu ihrer Herstellung und ihre Verwendung als Schädlingsbekämpfungsmittel.

Es ist bekannt, daß bestimmte 3-Methoxyacrylester, wie beispielsweise die Verbindung 2-(2-Methylphenyl)-3-methoxyacrylsäuremethylester fungizide Wirksamkeit besitzen (vergl. z. B. EP 178 826).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue substituierte Oximether der allgemeinen Formel (I),

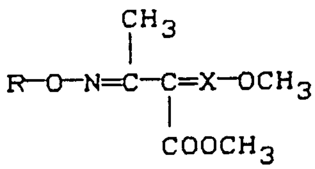

(I)

in welcher

R für jeweils unsubstituiertes oder substituiertes Alkyl, Cycloalkyl, Aryl oder Heterocyclyl steht und

X für Stickstoff oder eine CH-Gruppe steht,

gefunden.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Oximether der allgemeinen Formel (I),

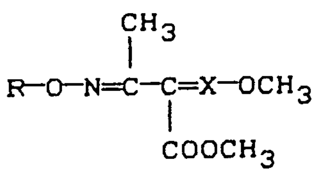

(I)

in welcher

R für jeweils unsubstituiertes oder substituiertes Alkyl, Cycloalkyl, Aryl oder Heterocycyl steht und

X für Stickstoff oder eine CH-Gruppe steht,

erhält, wenn man

(a) Hydroxyoximether der Formel (II),

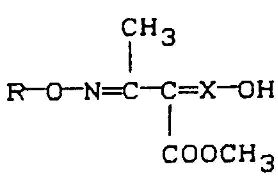

(II)

in welcher

R und X die oben angegebene Bedeutung haben, oder

deren Alkalimetall-Enolatsalze mit Methylierungsmitteln der Formel (III),

$CH_3$-E (III)

in welcher

E für eine elektronenanziehende Abgangsgruppe steht,

gegebenenfalls in Gegenwart eines Verdünnungsmittels und gegebenenfalls in Gegenwart eines Reaktionshilfsmittels umsetzt oder wenn man

(b) β-Ketocarbonsäureester der Formel (IV),

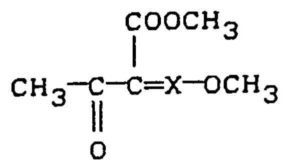

(IV)

in welcher

X die oben angegebene Bedeutung hat,

mit Hydroxylaminderivaten der Formel (V),

$R\text{-}O\text{-}NH_2$ (V)

in welcher

R die oben angegebene Bedeutung hat,

gegebenenfalls in Gegenwart eines Verdünnungsmittels und gegebenenfalls in Gegenwart eines Reaktionshilfsmittels umsetzt.

Schließlich wurde gefunden, daß die neuen substituierten Oximether der allgemeinen Formel (I) eine gute Wirksamkeit gegenüber Schädlingen, insbesondere gegenüber pflanzenschädigenden Pilzen besitzen.

Überraschenderweise zeigen die erfindungsgemäßen substituierten Oximether der allgemeinen Formel (I) eine erheblich bessere Wirksamkeit gegenüber pflanzenschädigenden Pilzen, als die aus dem Stand der Technik bekannten 3-Methoxyacrylester, wie beispielsweise die Verbindung 2-(2-Methylphenyl)-3-methoxyacrylsäuremethylester.

Die erfindungsgemäßen substituierten Oximether sind durch die Formel (I) allgemein definiert.

In den allgemeinen Formeln steht Alkyl, falls nicht anders definiert, für geradkettiges oder verzweigtes Alkyl mit vorzugsweise 1 bis 8, insbesondere 1 bis 6 und vor allem 1 bis 4 Kohlenstoffatomen, wobei Methyl, Ethyl, n- und i-Propyl, n-, i-, s- und t-Butyl, Pentyl und Hexyl beispielhaft und vorzugsweise genannt seien. Die Alkylreste können ihrerseits einen oder mehrere vorzugsweise 1 bis 3, insbesondere 1 oder 2 gleiche oder verschiedene Substituenten tragen. Als Substituenten seien beispielhaft und vorzugsweise genannt: Alkyl mit 1 bis 4 Kohlenstoffatomen, Alkoxy mit 1 bis 4 Kohlenstoffatomen, wie Methoxy, Ethoxy, n- und i-Propoxy und n-, i-, s- und t-Butoxy; Alkylthioalkyl mit 1 bis 4 Kohlenstoffatomen, wie Methylthio, Ethylthio, n- und i-Propylthio und n-, i-, s- und t-Butylthio sowie vorzugsweise gegebenenfalls substituiertes Aryl mit 6 bis 10 Kohlenstoffatomen, insbesondere gegebenenfalls substituiertes Phenyl.

Cycloalkyl steht im folgenden, falls nicht anders definiert, vorzugsweise für gegebenenfalls substituiertes und/oder insbesondere benzoannelliertes Cycloalkyl mit 3 bis 7, insbesondere 5 oder 6 Kohlenstoffatomen.

Aryl steht im folgenden, falls nicht anders definiert, vorzugsweise für gegebenenfalls substituiertes Aryl mit 6 bis 10 Kohlenstoffatomen, insbesondere gegebenenfalls substituiertes Phenyl.

Heterocyclyl steht im folgenden, falls nicht anders definiert, vorzugsweise für gegebenenfalls substituiertes und/oder insbesondere benzoannelliertes Heterocyclyl mit 2 bis 6, insbesondere 4 oder 5 Kohlenstoffatomen und 1 bis 3, insbesondere einem Heteroatom wie Stickstoff, Sauerstoff und Schwefel, insbesondere aber für Thiazolyl, Pyridinyl und Pyrimidinyl.

Die Arylreste, die Heterocyclylreste, der Phenylenteil von Cycloalkylresten sowie die Arylsubstituenten von Alkylresten der allgemeinen Formel können einen oder mehrere, vorzugsweise 1 bis 3, insbesondere 1 oder 2 gleiche oder verschiedene Substituenten tragen. Als Substituenten seien beispielhaft und vorzugsweise genannt: Halogen, wie Fluor, Chlor, Brom, Iod insbesondere Fluor, Chlor und Brom; Cyano; Nitro; Alkyl, Alkoxy und Alkylthio mit vorzugsweise 1 bis 4 Kohlenstoffatomen und vor allem Methyl, Ethyl, n- und i-Propyl, n-, i-, s- und t-Butyl, Methoxy, Ethoxy, n- und i-Propoxy und Methylthio; Halogenalkyl, Halogenalkoxy und Halogenalkylthio mit vorzugsweise 1 bis 4 Kohlenstoffatomen und 1 bis 9 gleichen oder verschiedenen Halogenatomen, insbesondere Trifluormethyl, Trifluormethoxy und Trifluormethylthio; Alkoxycarbonyl und Alkoximinoalkyl mit vorzugsweise 1 bis 4 Kohlenstoffatomen in den einzelnen Alkylteilen, insbesondere Methoxycarbonyl, Ethoxycarbonyl, Methoximinomethyl, Ethoximinomethyl, Methoximinoethyl und Ethoximinoethyl; gegebenenfalls mehrfach, insbesondere ein- bis dreifach, gleich oder verschieden durch Halogen, insbesondere Fluor, Chlor und Brom sowie Alkyl und Alkoxy mit 1 bis 4 Kohlenstoffatomen, insbesondere Methyl, Ethyl und Methoxy, substituiertes Phenyl.

Die Cycloalkylreste der allgemeinen Formel können im Cycloalkylteil eines gegebenenfalls benzoannellierten Cycloalkylrestes einen oder mehrere vorzugsweise 1 bis 3, insbesondere 1 oder 2 Substituenten tragen. Als Substituenten seien beispielhaft und vorzugsweise genannt: Halogen, wie Fluor, Chlor, Brom und Iod sowie Alkyl und Alkoxy mit vorzugsweise 1 bis 4 Kohlenstoffatomen, insbesondere Methyl, Ethyl, Methoxy und Ethoxy.

Bevorzugt sind Verbindungen der Formel (I), bei welchen

R für geradkettiges oder verzweigtes Alkyl mit 1 bis 8 Kohlenstoffatomen, für geradkettiges oder verzweigtes Alkoxyalkyl mit jeweils 1 bis 4 Kohlenstoffatomen in den einzelnen Alkylteilen, für geradkettiges oder verzweigtes Alkylthioalkyl mit jeweils 1 bis 4 Kohlenstoffatomen in den einzelnen Alkylteilen oder für unsubstituiertes oder einfach oder mehrfach gleich oder verschieden substituiertes Aralkyl mit 1 bis 6 Kohlenstoffatomen im geradkettigen oder verzweigten Alkylteil und 6 bis 10 Kohlenstoffatomen im Arylteil steht, wobei als Substituenten im Alkylteil infrage kommen: geradkettiges oder verzweigtes Alkyl mit 1 bis 4 Kohlenstoffatomen, jeweils geradkettiges oder verzweigtes Alkoxy und Alkylthio mit jeweils 1 bis 4 Kohlenstoffatomen und wobei als Substituenten im Arylteil infrage kommen: Halogen, Cyano, Nitro, jeweils geradkettiges oder verzweigtes Alkyl, Alkoxy und Alkylthio mit jeweils 1 bis 4 Kohlenstoffatomen, jeweils geradkettiges oder verzweigtes Halogenalkyl, Halogenalkoxy und Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie unsubstituiertes oder einfach oder mehrfach, gleich oder verschieden durch Halogen und/oder jeweils geradkettiges oder verzweigtes Alkyl oder Alkoxy jeweils mit 1 bis 4 Kohlenstoffatomen substituiertes Phenyl; oder für unsubstituiertes oder einfach oder mehrfach gleich oder verschieden substituiertes und/oder benzoannelliertes Cycloalkyl mit 3 bis 7 Kohlenstoffatomen steht, wobei als Substituenten im Cycloalkylteil infrage kommen: Halogen sowie jeweils geradkettiges oder verzweigtes Alkyl und Alkoxy mit jeweils 1 bis 4 Kohlenstoffatomen und wobei als Substituenten im gegebenenfalls anwesenden benzoannellierten Teil die obengenannten Arylsubstituenten infrage kommen; oder für unsubstituiertes oder einfach oder mehrfach, gleich oder verschieden substituiertes Aryl mit 6 bis 10 Kohlenstoffatomen steht, wobei als Substituenten die oben genannten Arylsubstituenten infrage kommen; oder für unsubstituiertes oder einfach oder mehrfach, gleich oder verschieden substituiertes und/oder benzoannelliertes Heterocyclyl mit 2 bis 6 Kohlenstoffatomen und 1 bis 3 Heteroatomen, insbesondere Stickstoff, Sauerstoff und/oder Schwefel steht, wobei als Substituenten im Heterocyclylteil und/oder im gegebenenfalls anwesenden benzoannellierten Teil die obengenannten Arylsubstituenten infrage kommen und

X für Stickstoff oder eine CH-Gruppe steht.

Besonders bevorzugt sind Verbindungen der Formel (I), bei welchen

R für geradkettiges oder verzweigtes Alkyl mit 1 bis 6 Kohlenstoffatomen oder für jeweils unsubstituiertes oder ein- bis dreifach, gleich oder verschieden substituiertes Phenyl oder Phenylalkyl mit 1 oder 2 Kohlenstoffatomen im geradkettigen oder verzweigten Alkylteil steht, wobei als Substituenten im Alkylteil von Phenylalkyl infrage kommen: Methyl, Ethyl, n- oder i-Propyl, n-, i-, s- oder t-Butyl, Methoxy, Ethoxy, Methylthio und Ethylthio und wobei als Substituenten von Phenyl bzw. im Phenylteil von Phenylalkyl infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl und unsubstituiertes oder ein- bis dreifach, gleich oder verschieden durch Fluor, Chlor, Brom, Methyl, Methoxy und/oder Ethyl substituiertes Phenyl; oder für einen unsubstituierten oder ein- bis fünffach, gleich oder verschieden substituierten Rest der Formel

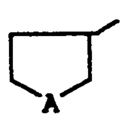 ; 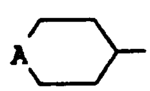 ; 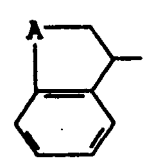 ; 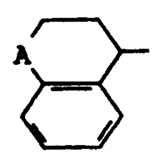

steht, wobei

A jeweils für Sauerstoff, Schwefel oder für eine $CH_2$-Gruppe steht und

wobei als Substituenten in den alicyclischen Ringen jeweils infrage kommen: Methyl, Ethyl, Methoxy und Ethoxy und wobei als Substituenten im Phenylenteil jeweils die oben genannten Phenylsubstituenten infrage kommen und

X für Stickstoff oder eine CH-Gruppe steht.

Ganz besonders bevorzugt sind Verbindungen der Formel (I), bei welchen

R für einen im Phenylteil unsubstituierten oder ein-bis dreifach, gleich oder verschieden substituierten Rest der Formel

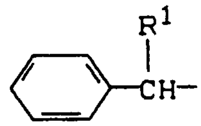

steht,

wobei

$R^1$ für Wasserstoff, Methyl, Ethyl, n- oder i-Propyl, für Methoxymethyl, Ethoxymethyl oder Methylthiomethyl steht und

wobei als Substituenten im Phenylteil jeweils infrage kommen: Fluor Chlor, Brom, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Methoxycarbonyl, Ethoxycarbonyl, Methoximinomethyl, Ethoximinomethyl, Methoximinoethyl, Ethoximinoethyl und unsubstistuiertes oder ein- bis dreifach, gleich oder verschieden durch Fluor, Chlor, Brom, Methyl, Methoxy und/oder Ethyl substituiertes Phenyl und

X für Stickstoff oder eine CH-Gruppe steht.

Ganz besonders bevorzugt sind daneben auch Verbindungen der Formel (I), bei welchen

R für einen im Phenylenteil unsubstituierten oder ein-bis dreifach, gleich oder verschieden substituierten Rest der Formel

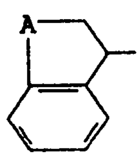

oder

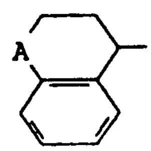

steht,

wobei

A jeweils für Sauerstoff, Schwefel oder eine $CH_2$-Gruppe steht und

wobei als Substituenten im Phenylenteil jeweils infrage kommen: Fluor, Chlor, Brom, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Methoxycarbonyl, Ethoxycarbonyl, Methoximinomethyl, Ethoximinomethyl, Methoximinoethyl, Ethoximinoethyl und unsubstituiertes oder ein- bis dreifach, gleich oder schieden durch Fluor, Chlor, Brom, Methyl, Methoxy und/oder Ethyl substituiertes Phenyl und

X für Stickstoff oder eine CH-Gruppe steht.

Im einzelnen seien außer den bei den Herstellungsbeispielen genannten Verbindungen die folgenden substituierten Oximether der allgemeinen Formel (I) genannt:

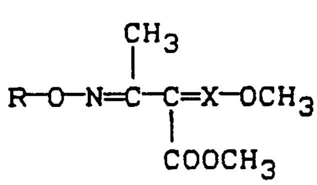

(I)

| R | X | R | X |
|---|---|---|---|
| 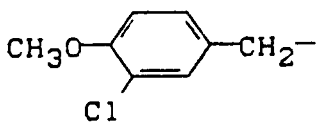 | CH | 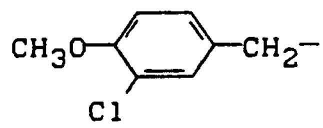 | N |
| 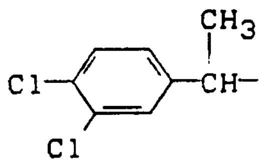 | CH | 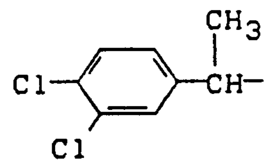 | N |
| 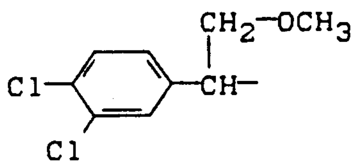 | CH | 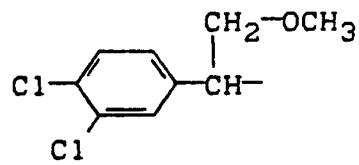 | N |
| 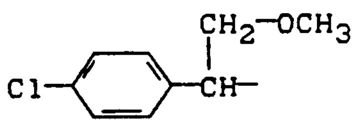 | CH | 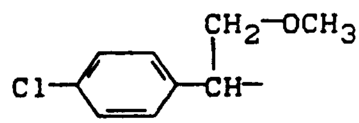 | N |
| 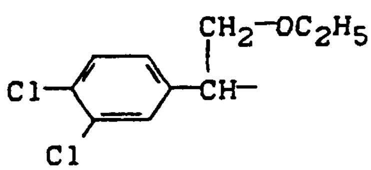 | CH | 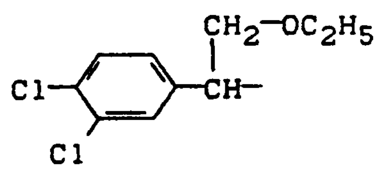 | N |

| R | X | R | X |
|---|---|---|---|
| 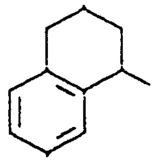 | CH | 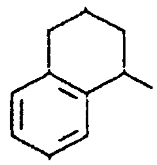 | N |
| 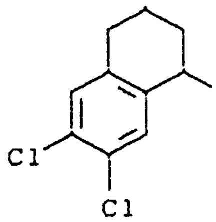 | CH | 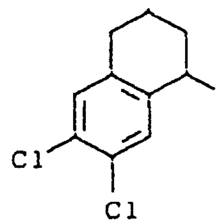 | N |
| 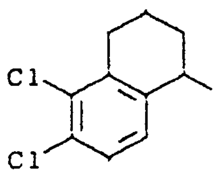 | CH | 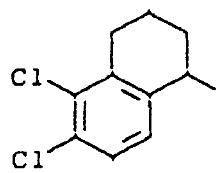 | N |
| 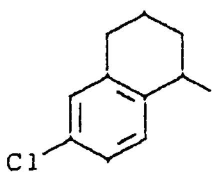 | CH | 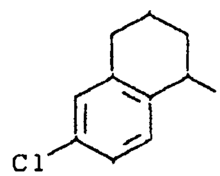 | N |
| 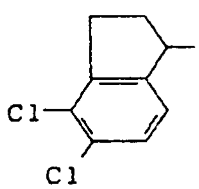 | CH | 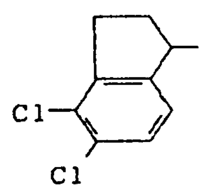 | N |
| 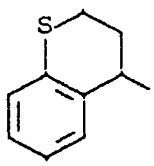 | CH | 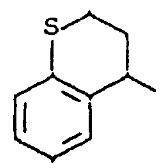 | N |
| 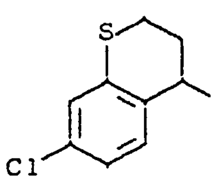 | CH | 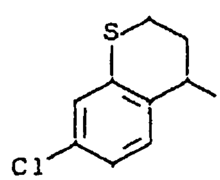 | N |
| 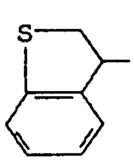 | CH | 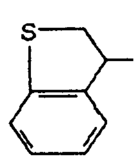 | N |

| R | X | R | X |
|---|---|---|---|
| 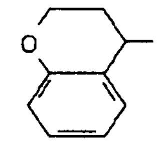 | CH | 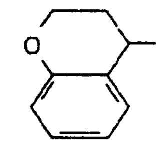 | N |
| 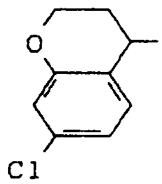 | CH | 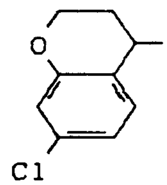 | N |
| 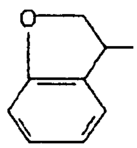 | CH | 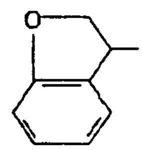 | N |
| 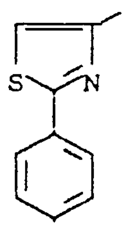 | CH | 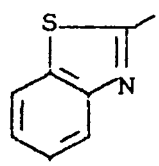 | CH |
| 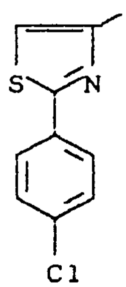 | CH | 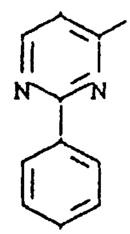 | CH |
| 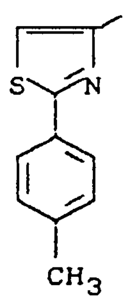 | CH | 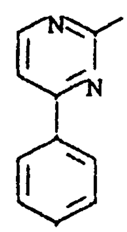 | CH |

Verwendet man beispielsweise 3-Benzyloximino-2-hydroxymethylidenbuttersäuremethylester und Dimethylsulfat als Ausgangsstoffe, so läßt sich der Reaktionsablauf des erfindungsgemäßen Verfahrens (a) durch das folgende Formelschema darstellen:

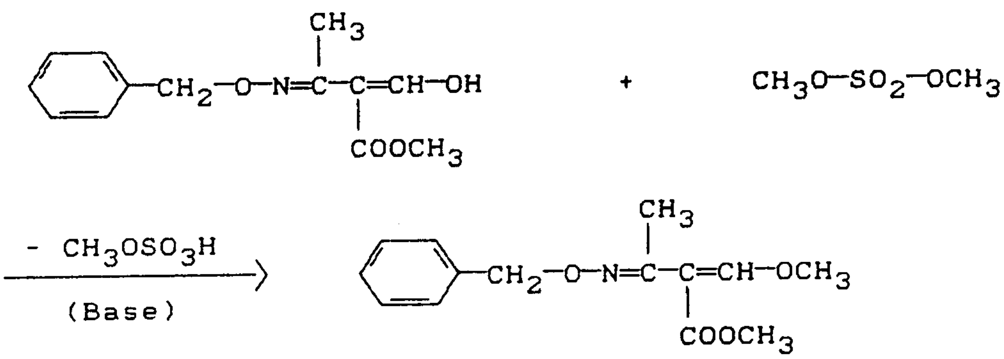


Verwendet man beispielsweise 2-Methoximino-3-oxobuttersäuremethylester und O-(1-Phenylethyl)-hydroxylamin als Ausgangsstoffe, so läßt sich der Reaktionsablauf des erfindungsgemäßen Verfahrens (b) durch das folgende Formelschema darstellen:

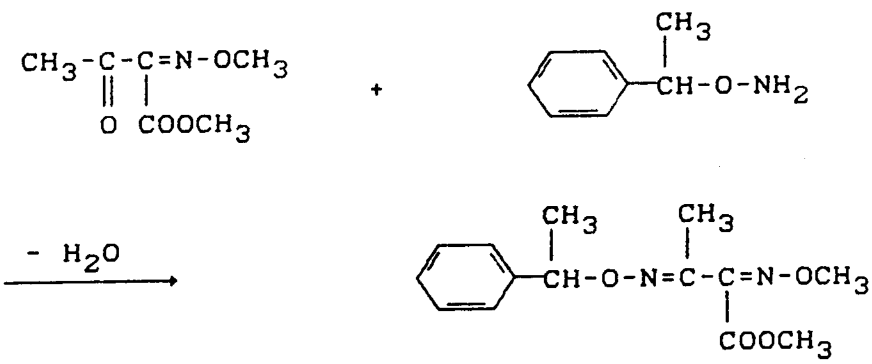


Die zur Durchführung des erfindungsgemäßen Verfahrens (a) als Ausgangsstoffe benötigten Hydroxyoximether sind durch die Formel (II) allgemein definiert. In dieser Formel (II) stehen R und X vorzugsweise für diejenigen Reste, die bereits im Zusammenhang mit der Beschreibung der erfindungsgemäßen Stoffe der Formel (I) als bevorzugt für diese Substituenten genannt wurden.

Die Hydroxyoximether sowie deren Alkalimetall-Enolatsalze der Formel (II) sind noch nicht bekannt und ebenfalls Gegenstand der Erfindung; man erhält sie, wenn man Acetessigsäuremethylester der Formel (VI)

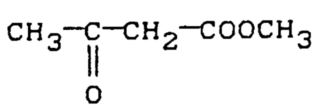
(VI)

mit Hydroxylaminderivaten der Formel (V),

$R\text{-}O\text{-}NH_2$ (V)

in welcher

R die oben angegebene Bedeutung hat,

gegebenenfalls in Gegenwart eines Verdünnungsmittels wie beispielsweise Toluol und gegebenenfalls in Gegenwart eines wasserbindenden Reaktionshilfsmittels wie beispielsweise eines Molekularsiebes bei Temperaturen zwischen 0 °C und 180 °C umsetzt [vgl. hierzu auch die Reaktionsbedingungen zur Durchführung des analogen erfindungsgemäßen Verfahrens (b)] und anschließend die so erhältlichen Oximinobuttersäuremethylester der Formel (VII),

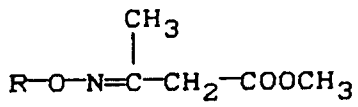

(VII)

in welcher

R die oben angegebenen Bedeutung hat,

entweder

(α) zunächst mit Dimethylformamiddimethylacetal gegebenenfalls in Gegenwart eines Verdünnungsmittels wie beispielsweise Dimethylformamid oder Toluol bei Temperaturen zwischen 50 °C und 180 °C umsetzt zu den Enaminen der Formel (VIII),

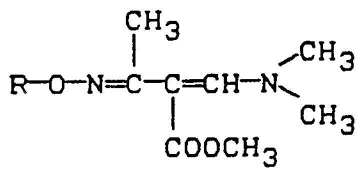

(VIII)

in welcher

R die oben angegebene Bedeutung hat,

und diese in einer anschließenden Reaktionsstufe (oder ohne Isolierung direkt im Eintopfverfahren) mit einer verdünnten wässrigen Mineralsäure, wie beispielsweise Salzsäure, gegebenenfalls in Gegenwart eines Verdünnungsmittels wie beispielsweise Dimethylformamid oder Aceton bei Temperaturen zwischen 20 °C und 100 °C umsetzt oder

(β) mit Ameisensäuremethylester gegebenenfalls in Gegenwart eines Verdünnungsmittels wie beispielsweise Dimethylformamid oder Toluol sowie gegebenenfalls in Gegenwart eines basischen Reaktionshilfsmittels wie beispielsweise Natriumhydrid bei Temperaturen zwischen 0 °C und 40 °C umsetzt oder

(γ) mit einem Alkylnitrit wie beispielsweise Isopentylnitrit gegebenenfalls in Gegenwart eines Verdünnungsmittels wie beispielsweise Methanol und gegebenenfalls in Gegenwart eines Reaktionshilfsmittels wie beispielsweise Natriummethylat bei Temperaturen zwischen 0 °C und 60 °C nitrosiert

Oximinobuttersäuremethylester der Formel (VII) und Enamine der Formel (VIII) sind noch nicht bekannt und ebenfalls Gegenstand der Erfindung.

Die zur Durchführung des erfindungsgemäßen Verfahrens (a) als Ausgangsstoffe benötigten Methylierungsmittel sind durch die Formel (III) allgemein definiert. In dieser Formel (III) steht E vorzugsweise für eine bei Methylierungsmitteln übliche Abgangsgruppe, wie beispielsweise Brom, Iod, Methoxysulfonyloxy oder p-Toluolsulfonyloxy.

Die Methylierungsmittel der Formel (III) sind allgemein bekannte Verbindungen der organischen Chemie.

Die zur Durchführung des erfindungsgemäßen Verfahrens (b) als Ausgangsstoffe benötigten β-Ketocarbonsäureester sind durch die Formel (IV) allgemein definiert und werden z.B. in PCT Int. Appl. WO 86/1202; US 4.555.517; J. Chem Soc., Perkin Trans. 1, 464 - 471 [1979]; J. Antibiotic. 34, 160 - 170 [1981]; JP 53/34795; DE-OS 2805655; CH 637141 beschrieben oder sind in Analogie zu bekannten Verfahren erhältlich.

Die zur Durchführung des erfindungsgemäßen Verfahrens (b) und zur Synthese der Vorprodukte der Formeln (II), (VII) und (VIII) als Ausgangsstoffe benötigten Hydroxylaminderivate sind durch die Formel (V) allgemein definiert.

In dieser Formel (V) steht R vorzugsweise für diejenigen Reste, die bereits im Zusammenhang mit der Beschreibung der erfindungsgemäßen Stoffe der Formel (I) als bevorzugt für diese Substituenten genannt wurden.

Die Hydroxylaminderivate der Formel (V) sind allgemein bekannte Verbindungen der organischen Chemie oder erhältlich in Analogie zu allgemein bekannten Verfahren (vgl. z. B. GB 1042191; Tetrahedron Lett. 23, 2955 - 2956 [1982]; DE-OS 3615473)

Als Verdünnungsmittel zur Durchführung des erfindungsgemäßen Verfahrens (a) kommen inerte organische Lösungsmittel infrage. Hierzu gehören insbesondere aliphatische alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan Dichlormethan, Chloroform, Tetrachlorkohlenstoff, Ether, wie Diethylether, Dioxan, Tetrahydrofuran oder Ethylenglykoldimethyl oder -diethylether, Ketone, wie Aceton oder Butanon, Nitrile, wie Acetonitril oder Propionitril, Amide, wie Dimethylformaid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (a) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyl-dimethyl-ammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxyethoxy)-ethyl]-amin.

Das erfindungsgemäße Verfahren (a) wird vorzugsweise in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen - 20 °C und 200 °C, vorzugsweise bei Temperaturen zwischen 0 °C und 180 °C.

Zur Durchführung des erfindungsgemäßen Verfahrens (a) setzt man pro Mol an Hydroxyoximether der Formel (II) oder eines entsprechenden Alkalimetall-Enolatsalzes im allgemeinen 1.0 bis 5.0 Mol, vorzugsweise 1.0 bis 1.5 Mol an Methylierungsmittel der Formel (III) und gegebenenfalls 1.0 bis 5.0 Mol, vorzugsweise 1.0 bis 2.0 Mol an Reaktionshilfsmittel ein.
Dabei ist es auch möglich die als Ausgangsverbindungen der Formel (II) zu verwendenden Hydroxyoximether der Formel (II) oder ihre entsprechenden Alkalimetall-Enolatsalze in einer vorgelagerten Reaktion direkt im Reaktionsgefäß herzustellen und ohne Isolierung aus dem Reaktionsgemisch heraus mit dem Methylierungsmittel der Formel (III) weiter umzusetzen (Eintopfreaktion). Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (b) kommen inerte organische Lösungsmittel oder deren Gemische mit Wasser infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid, Alkohole, wie Methanol, Ethanol, Propanol oder Butanol oder deren Gemische mit Wasser.

Das erfindungsgemäße Verfahren (b) kann gegebenenfalls in Gegenwart eines geeigneten wasserbindenden Reaktionshilfsmittels durchgeführt werden. Als solche kommen alle üblichen unter den Reaktionsbedingungen inerten und von den Reaktionsprodukten leicht abzutrennenden wasserbindenden Mittel infrage. Mit besonderem Vorzug entfernt man bei der Reaktion freiwerdendes Wasser durch azeotrope Destillation mit Hilfe eines Wasserabscheiders.

Die Reaktionstemperaturen können bei der Durchführung des erfindungsgemäßen Verfahrens (b) in einem größeren Bereich variiert werden. Im allgemeinen arbeitet man bei Temperaturen zwischen 20 °C und 200 °C, vorzugsweise bei Temperaturen zwischen 60 °C und 180 °C.

Zur Durchführung des erfindungsgemäßen Verfahrens (b) setzt man pro Mol an $\beta$-Ketocarbonsäureester der Formel (IV) im allgemeinen 1.0 bis 3.0 Mol, vorzugsweise 1.0 bis 1.2 Mol an Hydroxylaminderivat der Formel (V) und gegebenenfalls 1.0 bis 50.0 Mol, vorzugsweise 1.0 bis 5.0 Mol an Reaktionshilfsmittel ein.
Es ist auch möglich, die als Ausgangsverbindungen der Formel (V) zu verwendenden Hydroxylaminderivate der Formel (V) in Form von geeigneten Säureadditionssalzen, wie beispielsweise Hydrochloriden einzusetzen. In diesem Fall arbeitet man üblicherweise in Gegenwart von äquivalenten Mengen eines geeigneten Säurebindemittels. Als solche verwendet man vorzugsweise Alkalimetallcarbonate oder -acetate, wie beispielsweise Natriumcarbonat, Kaliumcarbonat oder Natriumacetat.

Die Reaktionsdurchführung, Aufarbeitung und Isolierung der Reaktionsprodukte erfolgt nach allgemein üblichen Methoden (vgl. auch die Herstellungsbeispiele).

Die mit Hilfe der erfindungsgemäßen Verfahren (a) oder (b) erhältlichen substituierten Oximether der Formel (I) fallen in der Regel als Stereoisomerengemische unterschiedlicher Zusammensetzung an. Diese Stereoisomerengemische können entweder als solche erfindungsgemäß verwendet werden oder mit Hilfe üblicher Trennmethoden in die einzelnen Komponenten aufgetrennt werden. Als Trennmethoden kommen dabei insbesondere Flüssigchromatographie oder Hochvakuumdestillation infrage.

Die Charakterisierung der Produkte erfolgt mit Hilfe des Schmelzpunktes oder der Protonen-Kernresonanzspektren.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger der Netzfleckenkrankheit der Gerste (Pyrenophora teres) oder gegen den Erreger der Braunspelzigkeit des Weizens (Leptosphaeria nodorum) oder gegen den Erreger des echten Getreidemehltaus (Erysiphe graminis) sowie zur Bekämpfung von Reiskrankheiten, wie beispielsweise gegen den Erreger der Reisfleckenkrankheit (Pyricularia oryzae) oder gegen den Erreger der Reisstengelkrankheit (Pellicularia sasakii) oder zur Bekämpfung von Krankheiten im Obst- und Gemüseanbau, wie beispielsweise gegen den Erreger des Apfelschorfes (Venturia inaequalis) oder gegen Oomyceten einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele:

Beispiel 1:

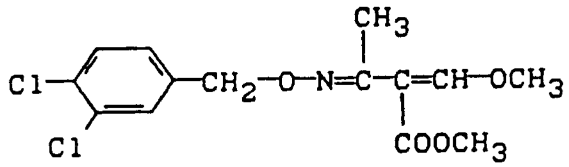

(Verfahren a)

Zu 7.1 g (0.0223 Mol) 3-(3,4-Dichlorbenzyloximino)-2-hydroxymethylidenbuttersäuremethylester in 23 ml Dimethylformamid gibt man nacheinander 6.2 g (0.0449 Mol) Kaliumcarbonat und dann tropfenweise unter Eiskühlung 2.9 g (0.023 Mol) Dimethylsulfat, rührt anschließend 2 Stunden bei Raumtemperatur, gießt dann die Reaktionsmischung in 140 ml Eiswasser, extrahiert dreimal mit jeweils 70 ml Diethylether, wäscht die vereingten organischen Phasen mit 140 ml Wasser, trocknet über Natriumsulfat und engt im Vakuum ein.

Man erhält 6.8 g (92 % der Theorie) an 3-(3,4-Dichlorbenzyloximino)-2-methoxymethylidenbuttersäuremethylester als Isomerengemisch, welches sich durch Chromatographie an Kieselgel (Laufmittel: Diethylether/Petrolether 1:1) in 2 Fraktionen auftrennen läßt und im Hochvakuum destilliert werden kann.

Isomeres A: 2,7 g (36 % der Theorie)

$^1$H-NMR ($CDCl_3$/Tetramethylsilan):

$\delta$ = 2.02 (3H); 3,71 (3H); 3.88 (3H); 5.02 (2H); 7.11 (1H); 7.33 - 7.42 (2H); 7.31 (1H) ppm.

Isomeres B: 3.7 g (49 % der Theorie)

$^1$H-NMR ($CDCl_3$/Tetramethylsilan):

$\delta$ = 2.04 (3H); 3.72 (3H); 3.87 (3H); 5.12 (2H); 7.2 (1H); 7.33 - 7.48 (2H); 7.44 (1H) ppm.

Alternatives Herstellungsverfahren (Verfahren a - Eintopfvariante):

Zu einer Suspension von 4.1 g (0.137 Mol) Natriumhydrid (80prozentig in Paraffin) in 70 ml Dimethylformamid tropft man unter Rühren bei Raumtemperatur eine Lösung von 20 g 3-(3,4-Dichlorbenzyloximino)-buttersäuremethylester in 41.4 g (0.689 Mol) Ameisensäuremethylester, rührt anschließend 12 Stunden bei Raumtemperatur und gibt dann bei 0 °C unter Rühren und Kühlung nacheinander tropfenweise 6.6 g (0.069 Mol) Methansulfonsäure, anschließend 9.5 g (0.069 Mol) Kaliumcarbonat und schließlich ebenfalls tropfenweise unter Rühren und Kühlung 8.7 g (0.069 Mol) Dimethylsulfat zu. Nach beendeter Zugabe rührt man 2 Stunden bei Raumtemperatur, gießt dann die Reaktionsmischung in Wasser, extrahiert mehrfach mit Ether, trocknet über Natriumsulfat, engt im Vakuum ein und reinigt den Rückstand durch Chromatographie an Kieselgel (Laufmittel: Ether/Petrolether 1:1).

Man erhält 10.1 g (44 % der Theorie) an 3-(3,4 Dichlorbenzyloximino)-2-methoxymethylidenbuttersäuremethylester als Isomerengemisch.

Beispiel 2:

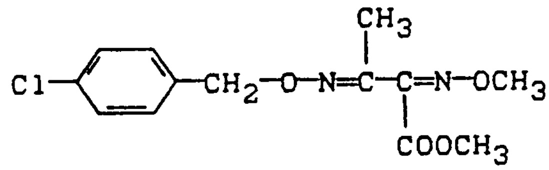

(Verfahren b)

4.3 g (0.027 Mol) 2-Methoximinoacetessigsäuremethylester (vgl. z. B. DE-OS 28 05 590) werden zusammen mit 4.3 g (0.027 Mol) 0-(4-Chlorbenzyl)-hydroxylamin (vgl. z. B. J. med. Chem. 10, 556 - 564 [1967]) in 30 ml Toluol 60 Minuten auf Rückflußtemperatur erhitzt. Anschließend wird das Lösungsmittel abdestilliert. Der Rückstand wird im Hochvakuum einer Kugelrohrdestillation unterzogen (Siedepunkt 100 °C bei 0.2 mbar).

Man erhält 5.5 g (68 % der Theorie) an 3-(4-Chlorbenzyloximino)-2-methoximino-buttersäuremethylester als Gemisch von zwei Isomeren A und B im Verhältnis 40 : 60.

Isomeres A:

$^1$H-NMR ($CDCl_3$/Tetramethylsilan):

$\delta$ = 2.09 (3H); 3.5 (3H); 3.99 (3H); 5.02 (2H); 7.2 -7.45 (4H) ppm.

Isomeres B:

$^1$H-NMR ($CDCl_3$/Tetramethylsilan):

$\delta$ = 2.05 (3H); 3.82 (3H); 3.99 (3H); 5.12 (2H), 7.2 - 7.45 (4H)

In entsprechender Weise und gemäß den allgemeinen Angaben zur Herstellung erhält man die folgenden substituierten Oximether der allgemeinen Formel (I);

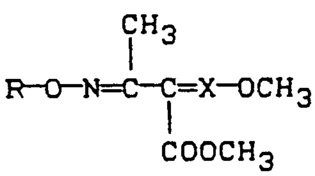

(I)

| Bsp.-Nr. | R | X | $^1$H-NMR*) |
|---|---|---|---|
| 3 | 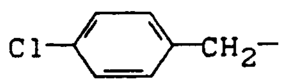 | CH | 2.0; 3.6; 3.8; 5.1; 7.2 - 7.4 |
| 4 | 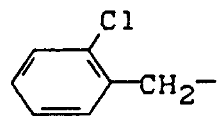 | CH | 2.1; 3.6; 3.85; 5.3; 7.1 - 7.5 |
| 5 | 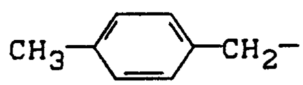 | CH | 2.2; 3.7; 3.8; 5.1; 7.1 - 7.5 |
| 6 | 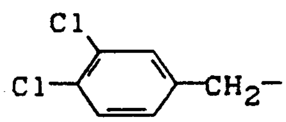 | N | 2.1; 3.7; 4.0; 5.1; 7.1 - 7.2 |
| 7 | 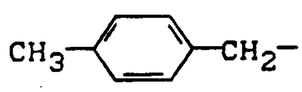 | N | 2.1; 3.6; 4.0; 5.05; 7.1 - 7.3 |
| 8 | 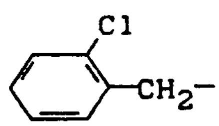 | N | 2.1; 3.7; 4.0; 5.25; 7.2 - 7.4 |
| 9 | 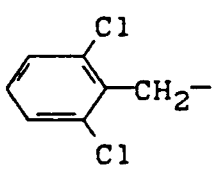 | N | 2.05; 3.6; 3.95; 5.4; 7.15 - 7.5 |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Herstellung der Ausgangsverbindungen:

Beispiel II-1:

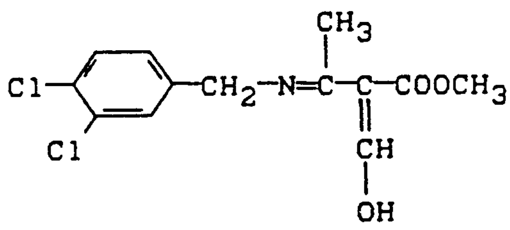

Zu 11.15 g (0.0323 Mol) 3-(3,4-Dichlorbenzyloximino)-2-dimethylaminomethyliden-buttersäuremethylester in 38 ml Dimethylformamid gibt man bei Raumtemperatur unter Kühlung und Rühren 18 ml (0.036 Mol) 2normale wässrige Salzsäure so, daß die Innentemperatur der Reaktionsmischung 35 ° C nicht überschreitet, läßt die Mischung 10 Minuten stehen, gießt sie dann in 200 nl Eiswasser, extrahiert 3 mal mit jeweils 100 ml Diethylether, wäscht die vereinigten organischen Phasen nacheinander mit 200 ml Wasser und 100 ml 2normaler Salzsäure, trocknet über Natriumsulfat engt im Vakuum ein und kristallisiert den Rückstand aus n-Hexan um.

Man erhält 7.8 g (76 % der Theorie) an 3-(3,4-Dichlorbenzyloximino)-2-hydroxymethylidenbuttersäuremethylester vom Schmelzpunkt 63 ° C - 65 ° C.

Beispiel II-2:

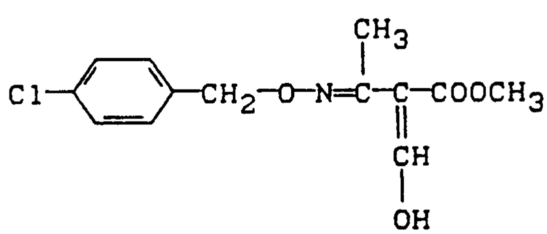

Zu einer Suspension von 1.2 g (0.04 Mol) Natriumhydrid in 20 ml Dimethylformamid gibt man bei Raumtemperatur tropfenweise unter Rühren eine Lösung von 5.1 g (0.02 Mol) an 3-(4-Chlorbenzyloximino)-buttersäuremethylester gelöst zu, rührt anschließend 4 Stunden bei Raumtemperatur, säuert dann mit 2normaler wässriger Salzsäure an, extrahiert mehrfach mit Essigsäureethylester, trocknet über Natriumsulfat, engt im Vakuum ein und reinigt den Rückstand durch Chromatographie an Kieselgel (Laufmittel: Cyclohexan/Essigester 2:1).

Man erhält 3.7 g (65 % der Theorie) als öliges Gemisch aus 2 Stereoismeren.

$^1$H-NMR ($CDCl_3$/Tetramethylsilan):

$\delta$ = 2.04; 2.3 (3H); 3.72; 3.8 (3H); 5.06 (2H) 7.2 - 7.4 (4H); 8.12 (1H); 11.8; 13.1 (1H) ppm.

In entsprechender Weise und gemäß den allgemeinen Angaben zur Herstellung erhält man die folgenden Hydroxyoximether der allgemeinen Formel (II):

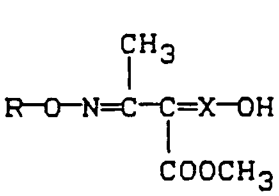

(II)

| Bsp.-Nr. | R | X | $^1$H-NMR*) |
|---|---|---|---|
| II-3 | 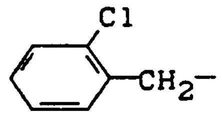 | CH | 2,34; 3,72; 5,20; 7,1 - 7,5; 8,17 |
| II-4 | 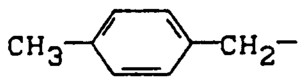 | CH | 2,30; 2,35; 3,70; 3,79; 5,0; 7,1 - 7,3; 8,22; 13,4 |
| II-5 | 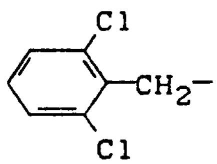 | CH | 2,27; 3,7; 5,38; 7,1 - 7,4; 8,13; 13,0 |
| II-6 | 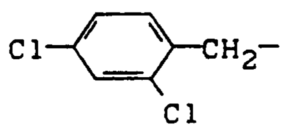 | CH | 2,3; 3,7; 5,16; 7,2 - 7,4; 8,1; 12,95 |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Beispiel VIII-1:

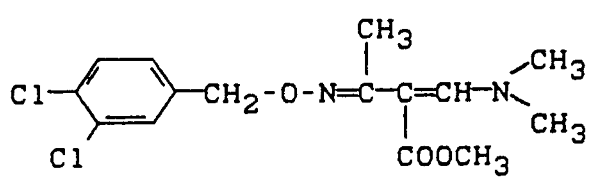

20 g (0.069 Mol) 3-(3,4-Dichlorbenzyloximino)-buttersäuremethylester werden zusammen mit 12.3 g (0.103 Mol) N,N-Dimethylformamiddimethylacetal in 35 ml Toluol 4 Stunden auf Rückflußtemperatur erhitzt, wobei freiwerdendes Methanol kontinuierlich abdestilliert wird. Anschließend wird im Vakuum eingeengt und der Rückstand im Hochvakuum einer Kugelrohrdestillation unterworfen (Kp 180 - 190 °C bei 0.1 mbar).

Man erhält 23 g (97 % der Theorie) an 3-(3,4-Dichlorbenzyloximino)-2-dimethylaminomethylidenbuttersäuremethylester, als öliges Gemisch aus 4 Stereoisomeren.

$^1$H-NMR ($CDCl_3$/Tetramethylsilan):

δ = 2.08 (3H); 2.78; 2.88 (6H); 3.68 (3H); 5.02; 5.08 (2H); 7.0 - 7.5 (4H) ppm.

In entsprechender Weise und gemäß den allgemeinen Angaben zur Herstellung erhält man die folgenden substituierten Enamine der allgemeinen Formel (VIII):

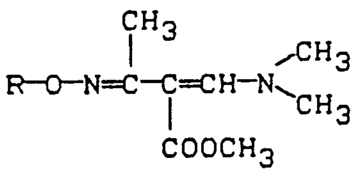

(VIII)

| Bsp.-Nr. | R | $^1$H-NMR*) |
|---|---|---|
| VIII-2 | 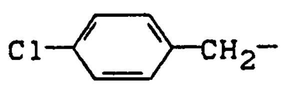 | 2,04; 2,75; 2,78; 3,64; 5,01; 5,1; 7,2 - 7,4 |
| VIII-3 | 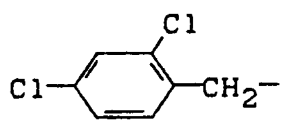 | 2,1; 2,44; 3,7; 5,12; 5,20; 7,1 - 7,5 |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als $\delta$-Wert in ppm.

Beispiel VII-1:

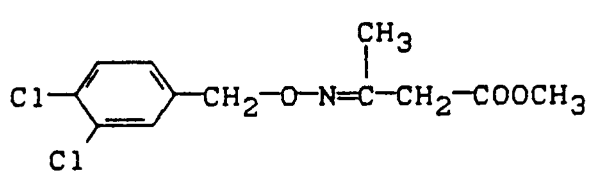

19.2 g (0.1 Mol) O-(3,4-Dichlorbenzyl)-hydroxylamin (vgl. z. B. DE-OS 31 16 888) und 11.6 g (0.1 Mol) Acetessigsäuremethylester werden in 100 ml Toluol 2 Stunden über einem Wasserabscheider auf Rückflußtemperatur erhitzt Zur Aufarbeitung wird abgekühlt im Vakuum eingeengt und der Rückstand im Hochvakuum destilliert.

Man erhält 21.6 g (74.4 % der Theorie) an 3-(3,4-Dichlorbenzyloximino)-buttersäuremethylester vom Siedepunkt 142 °C bis 152 °C bei 0.3 mbar.

In entsprechender Weise erhält man

Beispiel VII-2:

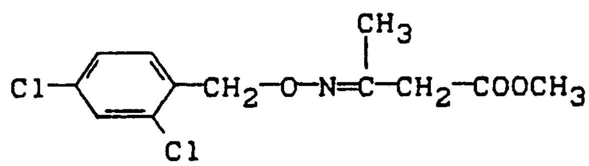

Kp 137 °C bei 0.5 mbar

Anwendungsbeispiele:

In dem folgenden Anwendungsbeispiel wurde die nachstehend aufgeführte Verbindung als Vergleichssubstanz eingesetzt:

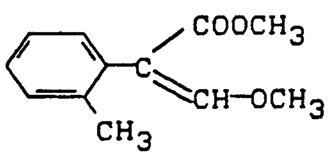

(A)

2-(2-Methylphenyl)-3-methoxyacrylsäuremethylester (bekannt aus EP 178 826)

Beispiel A

Pyrenophora teres-Test (Gerste) / protektiv / kurativ

Lösungsmittel: 100 Gewichtsteile Dimethylformamid
Emulgator: 0,25 Gewichtsteile Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Die Verbindung gemäß Herstellungsbeispiel 1 zeigt bei einer beispielhaften Wirkstoffkonzentration von 0,025 Gew.-% einen 100 %igen Wirkungsgrad. Die Verbindung des Standes der Technik zeigt keine Wirkung.

**Patentansprüche**

**1.** Substituierte Oximether der allgemeinen Formel (I)

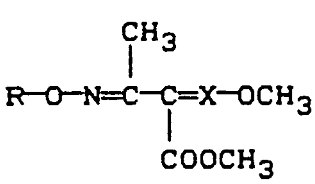

(I)

in welcher

R für jeweils unsubstituiertes oder substituiertes Alkyl, Cycloalkyl, Aryl oder Heterocyclyl steht und

X für Stickstoff oder eine CH-Gruppe steht.

**2.** Oximether der Formel (I) gemäß Anspruch 1, in welcher

R für geradkettiges oder verzweigtes Alkyl mit 1 bis 8 Kohlenstoffatomen, für geradkettiges oder verzweigtes Alkoxyalkyl mit jeweils 1 bis 4 Kohlenstoffatomen in den einzelnen Alkylteilen, für geradkettiges oder verzweigtes Alkylthioalkyl mit jeweils 1 bis 4 Kohlenstoffatomen in den einzelnen Alkylteilen oder für unsubstituiertes oder einfach oder mehrfach gleich oder verschieden substituiertes Aralkyl mit 1 bis 6 Kohlenstoffatomen im geradkettigen oder verzweigten Alkylteil und 6 bis 10 Kohlenstoffatomen im Arylteil steht, wobei als Substituenten im Alkylteil infrage kommen: geradkettig oder verzweigtes Alkyl mit 1 bis 4 Kohlenstoffatom jeweils gerad-kettiges oder verzweigtes Alkoxy und Alkylthio mit jeweils 1 bis 4 Kohlenstoffatomen und wobei als Substituenten im Arylteil infrage kommen: Halogen, Cyano, Nitro, jeweils geradkettiges oder verzweigtes Alkyl, Alkoxy und Alkylthio mit jeweils 1 bis 4 Kohlenstoffatomen, jeweils geradkettiges oder verzweigtes Halogenalkyl, Halogenalkoxy und Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie unsubstituiertes oder einfach oder mehrfach, gleich oder verschieden durch Halogen und/oder jeweils geradkettiges oder verzweigtes Alkyl oder Alkoxy jeweils mit 1 bis 4 Kohlenstoffatomen substituiertes Phenyl; oder für unsubstituiertes oder einfach oder mehrfach gleich oder verschieden substituiertes und/oder benzoannelliertes Cycloalkyl mit 3 bis 7 Kohlenstoffatomen steht, wobei als Substituenten im Cycloalkylteil infrage kommen: Halogen sowie jeweils geradkettiges oder verzweigtes Alkyl und Alkoxy mit jeweils 1 bis 4 Kohlenstoffatomen und wobei als Substituenten im benzoannellierten Teil die obengenannten Arylsubstituenten infrage kommen; oder für unsubstituiertes oder einfach oder mehrfach, gleich oder verschieden substituiertes Aryl mit 6 bis 10 Kohlenstoffatomen steht, wobei als Substituenten die oben genannten Arylsubstituenten infrage kommen; oder für unsubstituiertes oder einfach oder mehrfach, gleich oder verschieden substituiertes und/oder benzoannelliertes Heterocyclyl mit 2 bis 6 Kohlenstoffatomen und 1 bis 3 Heteroatomen, wobei als Substituenten im Heterocyclylteil und/oder im benzoannellierten Teil die obengenannten Arylsubstituenten infrage kommen und

X für Stickstoff oder eine CH-Gruppe steht.

**3.** Oximether der Formel (I) gemäß Anspruch 1, in welcher

R für geradkettiges oder verzweigtes Alkyl mit 1 bis 6 Kohlenstoffatomen oder für jeweils unsubstituiertes oder ein- bis dreifach, gleich oder verschieden substituiertes Phenyl oder Phenylalkyl mit 1 oder 2 Kohlenstoffatomen im geradkettigen oder verzweigten Alkylteil steht, wobei als Substituenten im Alkylteil von Phenylalkyl infrage kommen: Methyl, Ethyl, n- oder i-Propyl, n-, i-, s-oder t-Butyl, Methoxy, Ethoxy, Methylthio und Ethylthio und wobei als Substituenten von Phenyl bzw. im Phenylteil von Phenylalkyl infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl und unsubstituiertes oder ein- bis dreifach, gleich oder verschieden durch Fluor, Chlor, Brom, Methyl, Methoxy und/oder Ethyl substituiertes Phenyl; oder für einen unsubstituierten oder ein- bis fünffach, gleich oder verschieden substituierten Rest der Formel

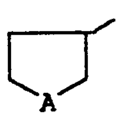 ; 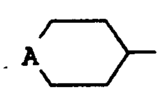 ; 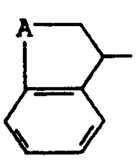 ; 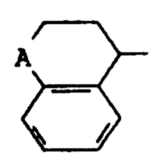

steht, wobei

A jeweils für Sauerstoff, Schwefel oder für eine $CH_2$-Gruppe steht und wobei als Substituenten in den alicyclischen Ringen jeweils infrage kommen: Methyl, Ethyl, Methoxy und Ethoxy und wobei als Substituenten im Phenylenteil jeweils die oben genannten Phenylsubstituenten infrage kommen und

X für Stickstoff oder eine CH-Gruppe steht.

**4.** Oximether der Formel (I) gemäß Anspruch 1, in welcher

R für einen im Phenylteil unsubstituierten oder ein-bis dreifach, gleich oder verschieden substituierten Rest der Formel

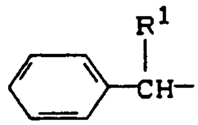

steht,

wobei

$R^1$ für Wasserstoff, Methyl, Ethyl, n- oder i-Propyl, für Methoxymethyl, Ethoxymethyl oder Methylthiomethyl steht und wobei als Substituenten im Phenylteil jeweils infrage kommen: Fluor, Chlor, Brom, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Methoxycarbonyl, Ethoxycarbonyl, Methoximinomethyl, Ethoximinomethyl, Methoximinoethyl, Ethoximinoethyl und unsubstituiertes oder ein- bis dreifach, gleich oder verschieden durch Fluor, Chlor, Brom, Methyl, Methoxy und/oder Ethyl substituierts Phenyl und

X für Stickstoff oder eine CH-Gruppe steht.

**5.** Oximether der Formel (I) gemäß Anspruch 1, in welcher

R für einen im Phenylenteil unsubstituierten oder ein- bis dreifach, gleich oder verschieden substituierten Rest der Formel

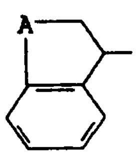

oder

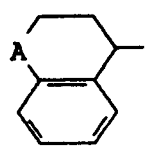

steht,

wobei

A jeweils für Sauerstoff, Schwefel oder eine $CH_2$-Gruppe steht und wobei als Substituenten im Phenylenteil jeweils infrage kommen: Fluor, Chlor, Brom, Methyl Ethyl, n- oder i-Propyl, n-, i-, s-oder t-Butyl, Methoxy, Ethoxy, n- oder i-Propoxy, Methylthio, Trifluormethyl, Trifluormethoxy, Methoxycarbonyl, Ethoxycarbonyl, Methoximinomethyl, Ethoximinomethyl, Methoximinoethyl, Ethoximinoethyl und unsubstituiertss oder ein- bis dreifach, gleich oder verschieden durch Fluor, Chlor, Brom, ttethyl, Methoxy und/oder Ethyl substituiertes Phenyl und

X für Stickstoff oder eine CH-Gruppe steht.

6. Verfahren zur Herstellung von Oximethern der allgemeinen Formel (I),

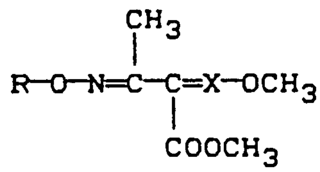

(I)

in welcher
R für jeweils unsubstituiertes oder substituiertes Alkyl, Cycloalkyl, Aryl oder Heterocyclyl steht und
X für Stickstoff oder eine CH-Gruppe steht,
dadurch gekennzeichnet, daß man
(a) Hydroxyoximether der Formel (II),

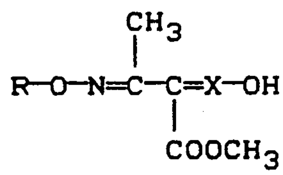

(II)

in welcher
R und X die oben angegebene Bedeutung haben, oder
deren Alkalimetall-Enolatsalze mit Methylierungsmitteln der Formel (III),

$CH_3$-E (III)

in welcher
E für eine elektronenanziehende Abgangsgruppe steht,
gegebenenfalls in Gegenwart eines Verdünnungsmittels und gegebenenfalls in Gegenwart eines Reaktionshilfsmittels umsetzt oder
(b) β-Ketocarbonsäureester der Formel (IV),

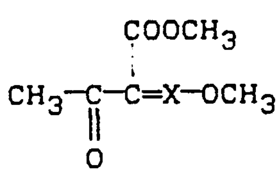

(IV)

in welcher
X die oben angegebene Bedeutung hat,
mit Hydroxylaminderivaten der Formel (V),

R-O-$NH_2$ (V)

in welcher
R die oben angegebene Bedeutung hat,
gegebenenfalls in Gegenwart eines Verdünnungsmittels und gegebenenfalls in Gegenwart eines Reaktionshilfsmittels umsetzt.

7. Schädlingsbekämpfungsmittel, gekennzeichnet durch einen Gehalt an mindestens einem Oximether der Formel (I) nach den Ansprüchen 1 bis 6.

**8.** Verwendung von Oximethern der Formel (I) nach den Ansprüchen 1 bis 6 zur Bekämpfung von Schädlingen.

**9.** Verfahren zur Bekämpfung von Schädlingen, dadurch gekennzeichnet, daß man Oximether der Formel (I) nach den Ansprüchen 1 bis 6 auf Schädlinge und/oder ihren Lebensraum einwirken läßt.

**10.** Verfahren zur Herstellung von Schädlingsbekämpfungsmitteln, dadurch gekennzeichnet, daß man Oximether der Formel (I) nach den Ansprüchen 1 bis 6 mit Streckmitteln und/oder oberflächenaktiven Mitteln vermischt.

**11.** Hydroximether der allgemeinen Formel (II)

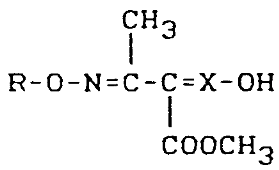

(II)

in welcher

R für jeweils unsubstituiertes oder substituiertes Alkyl, Cycloalkyl, Aryl oder Heterocyclyl steht und

X für Stickstoff oder eine CH-Gruppe steht,

ausgenommen Verbindungen, in denen X für Stickstoff steht und R $C_1$- bis $C_3$-Alkyl bedeutet.

**12.** Oximbuttersäuremethylester der allgemeinen Formel (VII)

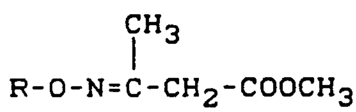

(VII)

in welcher

R für jeweils unsubstituiertes oder substituiertes Alkyl, Cycloalkyl, Aryl oder Heterocyclyl steht.

**13.** Enamine der allgemeinen Formel (VIII)

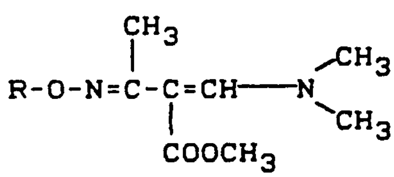

(VIII)

in welcher

R für jeweils unsubstituiertes oder substituiertes Alkyl, Cycloalkyl Aryl oder Heterocyclyl steht.

## Claims

**1.** Substituted oxime ethers of the general formula (I)

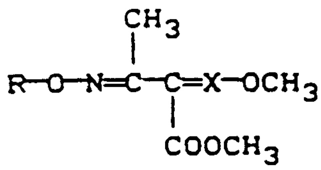

(I)

in which

R represents in each case unsubstituted or substituted alkyl, cycloalkyl, aryl or heterocyclyl and

X represents nitrogen or a CH group.

**2.** Oxime ethers of the formula (I) according to Claim 1, in which

R represents straight-chain or branched alkyl having 1 to 8 carbon atoms, or represents straight-chain or branched alkoxyalkyl having 1 to 4 carbon atoms in each of the individual alkyl moieties, or represents straight-chain or branched alkylthioalkyl having 1 to 4 carbon atoms in each of the individual alkyl moieties, or represents aralkyl which has 1 to 6 carbon atoms in the straight-chain or branched alkyl moiety and 6 to 10 carbon atoms in the aryl moiety and which is unsubstituted or monosubstituted or polysubstituted by identical or different substituents, suitable substituents in the alkyl moiety being: straight-chain or branched alkyl having 1 to 4 carbon atoms and in each case straight-chain or branched alkoxy and alkylthio, each having 1 to 4 carbon atoms, and suitable substituents in the aryl moiety being: halogen, cyano, nitro, in each case straight-chain or branched alkyl, alkoxy and alkylthio, each having 1 to 4 carbon atoms, in each case straight-chain or branched halogenoalkyl, halogenoalkoxy and halogenoalkylthio, each having 1 to 4 carbon atoms and 1 to 9 identical or different halogen atoms, in each case straight-chain or branched alkoxycarbonyl or alkoximinoalkyl, each having 1 to 4 carbon atoms in the individual alkyl moieties, and also phenyl which is unsubstituted or monosubstituted or polysubstituted by identical or different substituents from amongst halogen and/or in each case straight-chain or branched alkyl or alkoxy, each having 1 to 4 carbon atoms; or represents cycloalkyl which has 3 to 7 carbon atoms and which is benzo-fused and/or unsubstituted or monosubstituted or polysubstituted by identical or different substituents, suitable substituents in the cycloalkyl moiety being: halogen and in each case straight-chain or branched alkyl and alkoxy, each having 1 to 4 carbon atoms, and suitable substituents in the benzo-fused moiety being the abovementioned aryl substituents; or represents aryl which has 6 to 10 carbon atoms and which is unsubstituted or monosubstituted or polysubstituted by identical or different substituents, suitable substituents being the abovementioned aryl substituents; or represents heterocyclyl which has 2 to 6 carbon atoms and 1 to 3 hetero atoms and which is benzo-fused and/or unsubstituted or monosubstituted or polysubstituted by identical or different substituents, suitable substituents in the heterocyclyl moiety and/or in the benzo-fused moiety being the abovementioned aryl substituents, and

X represents nitrogen or a CH group.

**3.** Oxime ethers of the formula (I) according to Claim 1, in which

R represents straight-chain or branched alkyl having 1 to 6 carbon atoms, or represents phenyl or phenylalkyl having 1 or 2 carbon atoms in the straight-chain or branched alkyl moiety, each of which is unsubstituted or monosubstituted to trisubstituted by identical or different substituents, suitable substituents in the alkyl moiety of phenylalkyl being: methyl, ethyl, n- or i-propyl, n-, i-, s- or t-butyl, methoxy, ethoxy, methylthio and ethylthio, suitable substituents of phenyl or in the phenyl moiety of phenylalkyl being: fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy, trifluoromethylthio, methoxycarbonyl, ethoxycarbonyl, methoximinomethyl, ethoximinomethyl, methoximinoethyl, ethoximinoethyl and phenyl which is unsubstituted or monosubstituted to trisubstituted by identical or different substituents from amongst fluorine, chlorine, bromine, methyl, methoxy and/or ethyl; or represents a radical of the formula

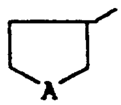 ; 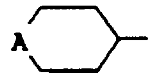 ; 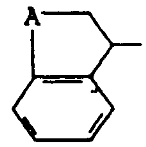 ; 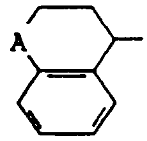

where

A in each case represents oxygen, sulphur or a $CH_2$ group,

which radical is unsubstituted or monosubstituted to pentasubstituted by identical or different substituents, suitable substituents in the alicyclic rings in each case being: methyl, ethyl, methoxy and ethoxy, and suitable substituents in the phenylene moiety in each case being the abovementioned phenyl substituents, and

X represents nitrogen or a CH group.

**4.** Oxime ethers of the formula (I) according to Claim 1, in which

R represents a radical of the formula

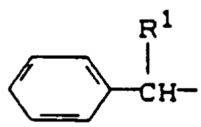

where

$R^1$ represents hydrogen, methyl, ethyl, n- or i-propyl, or represents methoxymethyl, ethoxymethyl or methylthiomethyl,

which radical is unsubstituted or monosubstituted to trisubstituted in the phenyl moiety by identical or different substituents, suitable substituents in the phenyl moiety in each case being: fluorine, chlorine, bromine, methyl, ethyl, n- or i-propyl, n-, i-, s- or t-butyl, methoxy, ethoxy, n- or i-propoxy, methylthio, trifluoromethyl, trifluoromethoxy, methoxycarbonyl, ethoxycarbonyl, methoximinomethyl, ethoximinomethyl, methoximinoethyl, ethoximinoethyl and phenyl which is unsubstituted or monosubstituted to trisubstituted by identical or different substituents from amongst fluorine, chlorine, bromine, methyl, methoxy and/or ethyl, and

X represents nitrogen or a CH group.

**5.** Oxime ethers of the formula (I) according to Claim 1, in which

R represents a radical of the formula

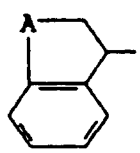 or 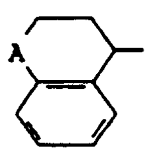

where

A in each case represents oxygen, sulphur or a $CH_2$ group,

each of these radicals being unsubstituted or monosubstituted to trisubstituted in the phenylene moiety by identical or different substituents, suitable substituents in the phenylene moiety in each case being: fluorine, chlorine, bromine, methyl, ethyl, n- or i-propyl, n-, i-, s- or t-butyl, methoxy, ethoxy, n- or i-propoxy, methylthio, trifluoromethyl, trifluoromethoxy, methoxycarbonyl, ethoxycarbonyl, methoximinomethyl, ethoximinomethyl, methoximinoethyl, ethoximinoethyl and phenyl which is unsubstituted or monosubstituted to trisubstituted by identical or different substituents from amongst fluorine, chlorine, bromine, methyl, methoxy and/or ethyl, and

X represents nitrogen or a CH group.

**6.** Process for the preparation of oxime ethers of the general formula (I)

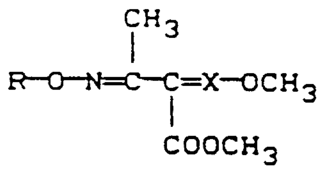

(I)

in which

R represents in each case unsubstituted or substituted alkyl, cycloalkyl, aryl or heterocyclyl and

X represents nitrogen or a CH group,

characterized in that

(a) hydroxyoxime ethers of the formula (II)

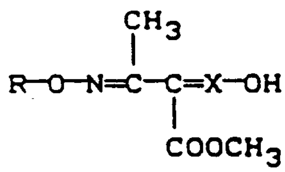

(II)

in which

R and X have the abovementioned meaning, or their alkali metal enolate salts, are reacted with methylating agents of the formula (III)

$CH_3$-E (III)

in which

E represents an electron-withdrawing leaving group,

if appropriate in the presence of a diluent and if appropriate in the presence of a reaction auxiliary, or

(b) $\beta$-ketocarboxylic esters of the formula (IV)

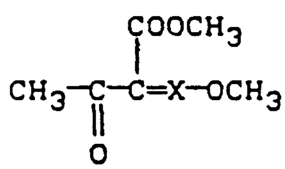

(IV)

in which

X has the abovementioned meaning

are reacted with hydroxylamine derivatives of the formula (V)

R-O-$NH_2$ (V)

in which

R has the abovementioned meaning,

if appropriate in the presence of a diluent and if appropriate in the presence of a reaction auxiliary.

**7.** Pesticides, characterized in that they contain at least one oxime ether of the formula (I) according to Claims 1 to 6.

**8.** Use of oxime ethers of the formula (I) according to Claims 1 to 6 for combating pests.

**9.** Method of combating pests, characterized in that oxime ethers of the formula (I) according to Claims 1 to 6 are applied to pests and/or their environment.

**10.** Process for the preparation of pesticides, characterized in that oxime ethers of the formula (I) according to Claims 1 to 6 are mixed with extenders and/or surface-active agents.

**11.** Hydroxime ethers of the general formula (II)

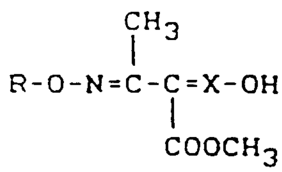

(II)

in which

R represents in each case unsubstituted or substituted alkyl, cycloalkyl, aryl or heterocyclyl and

X represents nitrogen or a CH group, with the exception of compounds in which X represents nitrogen and R denotes $C_1$- to $C_3$-alkyl.

**12.** Methyl hydroxyiminobutyrates, of the general formula (VII),

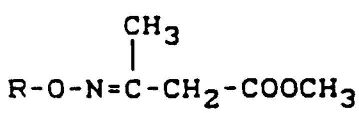

(VII)

in which

R represents in each case unsubstituted or substituted alkyl, cycloalkyl, aryl or heterocyclyl.

**13.** Enamines of the general formula (VIII)

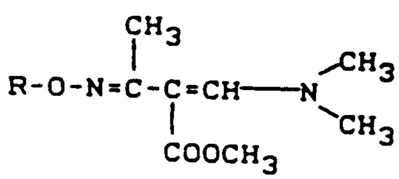

(VIII)

in which

R represents in each case unsubstituted or substituted alkyl, cycloalkyl, aryl or heterocyclyl.

**Revendications**

**1.** Oxime-éthers substitués répondant à la formule générale (I)

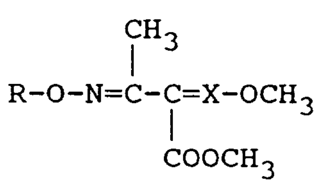

(I)

dans laquelle

R représente un groupe alkyle, un groupe cycloalkyle, un groupe aryle ou un groupe hétérocyclyle, chacun de ces groupes étant non substitué ou substitué, et

X représente un atome d'azote ou un groupe CH.

**2.** Oxime-éthers de formule (I) selon la revendication 1, dans laquelle

R représente un groupe alkyle à chaîne droite ou ramifiée contenant de 1 à 8 atomes de carbone, un groupe alcoxyalkyle à chaîne droite ou ramifiée contenant respectivement de 1 à 4 atomes de carbone dans les fractions alkyle individuelles, un groupe alkylthioalkyle à chaîne droite ou ramifiée contenant respectivement de 1 à 4 atomes de carbone dans les fractions alkyle individuelles, ou encore un groupe aralkyle non substitué ou substitué une ou plusieurs fois de manière identique ou différente contenant de 1 à 6 atomes de carbone dans la fraction alkyle à chaîne droite ou ramifiée et de 6 à 10 atomes de carbone dans la fraction aryle, en envisageant comme substituants dans la fraction alkyle : un groupe alkyle à chaîne droite ou ramifiée contenant de 1 à 4 atomes de carbone, un groupe alcoxy et un groupe alkylthio, chacun étant à chaîne droite ou ramifiée contenant chacun de 1 à 4 atomes de carbone, en envisageant comme substituants dans la fraction aryle : un atome d'halogène, un groupe cyano, un groupe nitro; un groupe alkyle, un groupe alcoxy et un groupe alkylthio, chacun de ces groupes étant à chaîne droite ou ramifiée et contenant chacun de 1 à 4 atomes de carbone; un groupe halogénalkyle, un groupe halogénalcoxy et un groupe halogénalkylthio, chacun de ces groupes étant à chaîne droite ou ramifiée et contenant chacun de 1 à 4 atomes de carbone et de 1 à 9 atomes d'halogène identiques ou différents; un groupe alcoxycarbonyle ou un groupe alcoximinoalkyle, chacun de ces groupes étant à chaîne droite ou ramifiée et contenant chacun de 1 à 4 atomes de carbone dans les fractions alkyle individuelles; ainsi qu'un groupe phényle non substitué ou substitué une ou plusieurs fois de manière identique ou différente par un atome d'halogène et/ou par un groupe alkyle ou par un groupe alcoxy, chacun étant à chaîne droite ou ramifiée et contenant chacun de 1 à 4 atomes de carbone; ou encore représente un groupe cycloalkyle non substitué ou substitué une ou plusieurs fois de manière identique ou différente et/ou un groupe cycloalkyle benzocondensé contenant de 3 à 7 atomes de carbone, en envisageant comme substituants dans la fraction cycloalkyle : un atome d'halogène; un groupe alkyle et un groupe alcoxy, chacun étant à chaîne droite ou ramifiée et contenant chacun de 1 à 4 atomes de carbone, en envisageant comme substituants dans la fraction benzocondensée les substituants du groupe aryle mentionnés ci-dessus, ou encore représente un groupe aryle non substitué ou substitué une ou plusieurs fois de manière identique ou différente contenant de 6 à 10 atomes de carbone, en envisageant comme substituants les substituants du groupe aryle mentionnés ci-dessus; ou encore représente un groupe hétérocyclyle non substitué ou substitué une ou plusieurs fois de manière identique ou différente et/ou un groupe hétérocyclyle benzocondensé contenant de 2 à 6 atomes de carbone et de 1 à 3 hétéroatomes, en envisageant comme substituants dans la fraction hétérocyclyle et/ou dans la fraction benzocondensée, les substituants du groupe aryle mentionnés ci-dessus, et

X représente un atome d'azote ou un groupe CH.

**3.** Oxime-éthers de formule (I) selon la revendication 1, dans laquelle

R représente un groupe alkyle à chaîne droite ou ramifiée contenant de 1 à 6 atomes de carbone ou bien un groupe phényle ou un groupe phénylalkyle chacun étant non substitué ou substitué de 1 à 3 fois de manière identique ou différente contenant 1 ou 2 atomes de carbone dans la fraction alkyle à chaîne droite ou ramifiée, en envisageant comme substituants dans la fraction alkyle du groupe phénylalkyle : un groupe méthyle, un groupe éthyle, un groupe n- ou i-propyle, un groupe n-, i-, s- ou t-butyle, un groupe méthoxy, un groupe éthoxy, un groupe méthylthio et un groupe éthylthio, et en envisageant comme substituants du groupe phényle ou dans la fraction phényle du groupe phénylalkyle : un atome de fluor, un atome de chlore, un atome de brome, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe n- ou i-propoxy, un groupe méthylthio, un groupe trifluorométhyle, un groupe trifluorométhoxy, un groupe trifluorométhylthio, un groupe méthoxycarbonyle, un groupe éthoxycarbonyle, un groupe méthoximinométhyle, un groupe éthoximinométhyle, un groupe méthoximinoéthyle et un groupe éthoximinoéthyle, ainsi qu'un

groupe phényle non substitué ou substitué de 1 à 3 fois de manière identique ou différente par un atome de fluor, un atome de chlore, un atome de brome, un groupe méthyle, un groupe méthoxy et/ou un groupe éthyle; ou représente un radical non substitué ou substitué de 1 à 5 fois de manière identique ou différente répondant aux formules

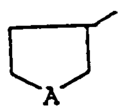 ; 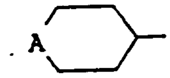 ; 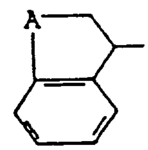 ; 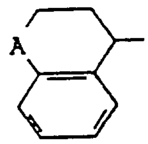

dans lesquelles

A représente, respectivement, un atome d'oxygène, un atome de soufre ou un groupe $CH_2$ et en envisageant comme substituants dans les noyaux alicycliques respectivement : un groupe méthyle, un groupe éthyle, un groupe méthoxy et un groupe éthoxy, et en envisageant comme substituants dans la fraction phénylène, respectivement, les substituants du groupe phényle mentionnés ci-dessus, et

X représente un atome d'azote ou un groupe CH.

**4.** Oxime-éthers de formule (I) selon la revendication 1, dans laquelle

R représente un radical non substitué dans la fraction phényle ou encore substitué de 1 à 3 fois de manière identique ou différente répondant à la formule

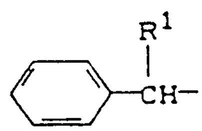

dans laquelle

$R^1$ représente un atome d'hydrogène, un groupe méthyle, un groupe éthyle, un groupe n- ou i-propyle, un groupe méthoxyméthyle, un groupe éthoxyméthyle ou un groupe méthylthiométhyle et

en envisageant comme substituants dans la fraction phényle, respectivement : un atome de fluor, un atome de chlore, un atome de brome, un groupe méthyle, un groupe éthyle, un groupe n- ou i-propyle, un groupe n-, i-, s- ou t-butyle, un groupe méthoxy, un groupe éthoxy, un groupe n- ou i-propoxy, un groupe méthylthio, un groupe trifluorométhyle, un groupe trifluorométhoxy, un groupe méthoxycarbonyle, un groupe éthoxycarbonyle, un groupe méthoximinométhyle, un groupe éthoximinométhyle, un groupe méthoximinoéthyle, un groupe éthoximinoéthyle, ainsi qu'un groupe phényle non substitué ou substitué de 1 à 3 fois de manière identique ou différente par un atome de fluor, un atome de chlore, un atome de brome, un groupe méthyle, un groupe méthoxy et/ou un groupe éthyle, et

X représente un atome d'azote ou un groupe CH.

**5.** Oxime-éthers de formule (I) selon la revendication 1, dans laquelle

R représente un radical non substitué dans la fraction phénylène ou encore substitué de 1 à 3 fois de manière identique ou différente répondant aux formules

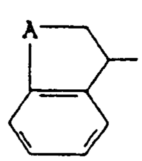 ou 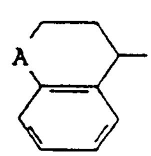

dans lesquelles

A représente, respectivement, un atome d'oxygène, un atome de soufre ou un groupe $CH_2$, en envisageant comme substituants dans la fraction phénylène, respectivement : un atome de fluor, un atome de chlore, un atome de brome, un groupe méthyle, un groupe éthyle, un groupe n- ou i-propyle, un groupe n-, i-, s- ou t-butyle, un groupe méthoxy, un groupe éthoxy, un groupe n- ou i-propoxy, un groupe méthylthio, un groupe trifluorométhyle, un groupe trifluorométhoxy, un groupe méthoxycarbonyle, un groupe éthoxycarbonyle, un groupe méthoximinométhyle, un groupe éthoximinométhyle, un groupe méthoximinoéthyle, un groupe éthoximinoéthyle, ainsi qu'un groupe phényle non substitué ou substitué 1 à 3 fois de manière identique ou différente par un atome de fluor, un atome de chlore, un atome de brome, un groupe méthyle, un groupe méthoxy et/ou un groupe éthyle, et

X représente un atome d'azote ou un groupe CH.

**6.** Procédé pour la préparation d'oxime-éthers répondant à la formule générale (I)

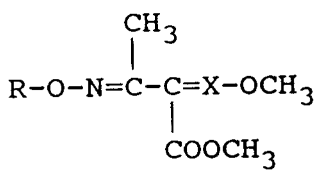

(I)

dans laquelle

R représente un groupe alkyle, un groupe cycloalkyle, un groupe aryle ou un groupe hétérocyclyle, chacun de ces groupes étant non substitué ou substitué, et

X représente un atome d'azote ou un groupe CH,

caractérisé en ce qu'on fait réagir

(a) des hydroxyoxime-éthers de formule (II)

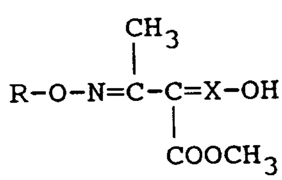

(II)

dans laquelle

R et X ont la signification indiquée ci-dessus, ou

leurs sels énolates de métaux alcalins avec des agents de méthylation de formule (III)

$CH_3$-E (III)

dans laquelle

E représente un groupe qui se sépare, attirant les électrons,

éventuellement en présence d'un diluant et éventuellement en présence d'un adjuvant réactionnel ou

(b) des esters $\beta$-cétocarboxyliques de formule (IV)

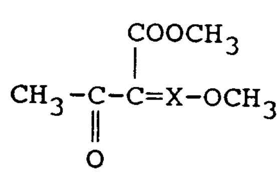

(IV)

dans laquelle

X a la signification indiquée ci-dessus,

avec des dérivés d'hydroxylamine de formule (V)

$R\text{-}O\text{-}NH_2$ (V)

dans laquelle

R a la signification indiquée ci-dessus, éventuellement en présence d'un diluant et éventuellement en présence d'un adjuvant réactionnel.

**7.** Agents de lutte contre les parasites, caractérisés par une teneur en au moins un oxime-éther de formule (I) selon les revendications 1 à 6.

**8.** Utilisation d'oxime-éthers de formule (I) selon les revendications 1 à 6 pour lutter contre les parasites.

**9.** Procédé pour lutter contre les parasites, caractérisé en ce qu'on laisse agir des oxime-éthers de formule (I) selon les revendications 1 à 6, sur des parasites et/ou sur leur biotope.

**10.** Procédé pour la préparation d'agents de lutte contre les parasites, caractérisé en ce qu'on mélange des oxime-éthers de formule (I) selon les revendications 1 à 6, avec des diluants et/ou avec des agents tensioactifs.

**11.** Hydroxime-éthers répondant à la formule générale (II)

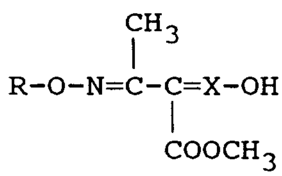

(II)

dans laquelle

R représente un groupe alkyle, un groupe cycloalkyle, un groupe aryle ou un groupe hétérocyclyle, chacun de ces groupes étant non substitué ou substitué, et

X représente un atome d'azote ou un groupe CH,

à l'exclusion de composés dans lesquels X représente un atome d'azote et R désigne un groupe alkyle en $C_1$-$C_3$.

**12.** Ether méthylique de l'acide oxime-butyrique répondant à la formule générale (VII)

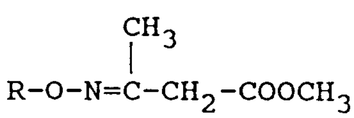

(VII)

dans laquelle

R représente un groupe alkyle, un groupe cycloalkyle, un groupe aryle ou un groupe hétérocyclyle, chacun de ces groupes étant non substitué ou substitué.

**13.** Enamines de formule générale (VIII)

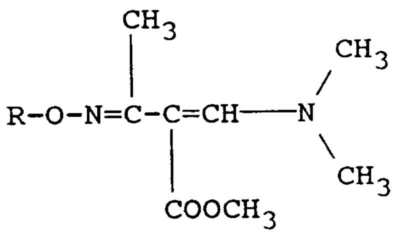

(VIII)

dans laquelle

R représente un groupe alkyle, un groupe cycloalkyle, un groupe aryle ou un groupe hétérocyclyle, chacun de ces groupes étant non substitué ou substitué.